# EUROPEAN PATENT APPLICATION

(11) **EP 1 970 705 A1**
(43) Date of publication of application: **17.09.2008**
(21) Application number: 07104212.1
(22) Date of filing: 15.03.2007
(51) Int. Cl.: G01N 33/543

(54) **Bead assisted analyte detection**

(71) Applicant: Koninklijke Philips Electronics N.V., 5621 BA Eindhoven (NL)
(72) Inventor: The designation of the inventor has not yet been filed
(74) Representative: van Wermeskerken, Stephanie Christine

(57) **Abstract**

The present invention relates to beads (7) for use in biotechnical applications such as biosensing. The beads (7) comprise at least one magnetic or magnetisable particle (34) and an organic moiety and are freeze-dried. The latter has the advantage that the reactivity of such particles is low, resulting in a longer shelf life. Furthermore, as the beads comprise a magnetic or magnetisable component, this allows accurately providing and positioning of the beads in a biosensor, e.g. by magnetic actuation. The magnetic or magnetisable component furthermore can be used as labels during biosensing. A method of manufacturing the beads, a method for analyzing sample fluid using the beads and a sensor system and method of manufacturing thereof also is disclosed.

## Description

### FIELD OF THE INVENTION

The present invention relates to the field of biosensors and diagnostics. More particularly, the present invention relates to processes for analyzing the presence of analytes in fluids, e.g. drugs of abuse in saliva, beads for use in such processes and methods for manufacturing such beads.

### BACKGROUND OF THE INVENTION

In a plurality of biotechnology applications, such as bio-sensing, use is made ofbio-reagents for biotechnological reactions. Such bio-reagents often are provided in a wet state. The shelf-life of bio-reagents in the field of biotechnology has long been a major problem. Deterioration of the bio-reagents during their storage results in a reduction of the quality of bio-sensing. Storage of the bioreagents in a dry state improves the shelf-life of bio-reagents.

US 2005/0069898 describes a method to manufacture lyophilized beads suitable for the amplification of a nucleic acid sequence. In this reference, the lyophilized beads are manufactured by a) forming a solution comprising mannitol, Bovine Serum Albumine (BSA) and oligonucleotide primers that can be labeled with radioisotopes, chemiluminescent moieties or fluorescent moieties b) dropping this solution onto a cryogenic liquid followed by c) freeze-drying the beads. The beads obtained are between 1 mm and 4.5 mm large. Such lyophilized beads have the advantage to extend the shelf-life of the oligonucleotide primers present within.

Analytes in a sample fluid can be detected by various means and recent developments enable the analysis to be performed on very small fluid samples. This miniaturization involves a real challenge since reagents having an extended shelf-life are still to be developed that could be a) easily and accurately placed in a well defined amount in chambers or channels of microfluidic analysis devices, which dimensions varies from a few tenth of µm to a few hundreds of µm, or in the microscopic wells of a sensor surface. For instance, in a competitive assay in which an analyte competes with an analyte analogue for a labeled probe, the sample fluid comprising the analyte is first mixed with a reagent comprising the labeled probe before contacting a sensor surface on which an analyte analogue is immobilized. The sensor is thereafter analyzed for the presence or absence of labels on its surface. Such assays require that a well-defined number of labeled probes and therefore also a well-defined amount of reagent is mixed with the sample fluid.

### SUMMARY OF THE INVENTION

It is an object of the present invention to provide devices and processes for analyzing the presence of analytes in fluids, methods of manufacturing such devices and methods using such devices. An advantage of embodiments of the present invention is the detection of an analyte in a sample fluid in a fast (e.g. within one minute) and reliable way. Another advantage of embodiments of the present invention is a long shelf life of the detection system. An advantage of embodiments of the present invention is an easy deposition of a well defined amount of reagent at precise places of a sensor or of a microfluidic system. Another advantage of embodiments of the present invention is to prevent reaction or mixing between the reagent and the sensor.

An advantage of embodiments of the present invention is the independent optimization of the reagent on one hand and the sensor on the other hand.

The above objective is accomplished by methods and products according to the present invention.

In a first aspect the present invention relates to a method for manufacturing a lyophilized bead, the method comprising providing a liquid mixture having a freezing temperature, the liquid mixture comprising one or more magnetic or magnetisable particles and an organic moiety, atomizing or spraying the liquid mixture into a fluid medium having a temperature lower than the freezing temperature to form the beads, and freeze-drying the beads. The liquid mixture can be a dispersion or a slurry. It is an advantage of embodiments of the present invention that beads are obtained wherein the magnetic or magnetisable particles enable an easy deposition of the lyophilized beads at precise places of e.g. a sensor. As an optional feature, spraying the liquid mixture may be adding droplets of the liquid mixture into the fluid medium to form frozen beads from the droplets. It is an advantage of embodiments of the present invention that droplets may be of a predetermined size and/or shape. Adding droplets may be jetting droplets in a droplet stream to the fluid medium, e.g. by means of a microjetting head. It is an advantage of embodiments of the present invention that beads can be made in an efficient way.

As an optional feature, adding droplets to the fluid medium may comprise monodisperse droplet formation and jetting. An advantage is that the beads produced are also substantially monodisperse.

The organic moiety may be a matrix, i.e. it can form a matrix for the magnetic particles. The organic moiety may optionally comprise a binding material to bind specifically to predetermined biological or biotechnological components or may include other optional components such as surfactants. The organic moiety may comprise one or more probes. Alternatively or in addition thereto, the organic moiety may comprise one or more primers. Alternatively or in addition thereto, the organic moiety may include vectors. Alternatively or in addition thereto, the organic moiety may comprise a therapeutic or diagnostic or a binding agent or a carbohydrate or a proteinaceous material or a nucleotide sequence or a combination of any of these. The carbohydrate may be a sugar.

As an optional feature, the method may comprise forming droplets of the liquid mixture using ink-jet technology. This is advantageous because ink-jet technology ensures a well-defined size and a relatively narrow size dispersity of the droplets and hence will control the size of lyophilized beads derived from the frozen droplets. This permits to indirectly dose the organic moiety such as probes with a relatively good precision by dosing the lyophilized beads with good precision.

As an optional feature, the fluid medium is a cryogenic liquid. This is advantageous because it ensures an efficient and very fast freezing of the droplet which prevents the formation of large solid crystals or liquid e.g. water (i.e. ice) crystals in the bead and permits to obtain a relatively amorphous bead structure and permits phase separation between the magnetic particles and the fluid during freezing.

As an optional feature, during atomizing or spraying of the liquid mixture functional or geometrical adjustment of the liquid mixture, e.g. functional or geometrical adjustment of droplets formed, may be performed. The latter may for example be performed after the liquid mixture has left the spraying nozzle and prior to its contact with the fluid medium. Functionally or geometrically influencing the liquid mixture may comprise magnetically influencing the liquid mixture or changing the shape of the droplets before they enter the freezing medium.

In a second aspect, the present invention relates to a bead for use in bio-sensing, the bead comprising one or more magnetic or magnetisable particles and an organic moiety, whereby the bead is lyophilized. The organic moiety may be a matrix. The organic moiety may optionally comprise a binding material to bind specifically to predetermined biological or biotechnological components or may comprise other components such as a surfactant. The organic moiety may comprise one or more probes. This is advantageous because despite the presence of reactive probes, beads can be obtained that have a low degree of reactivity, thus allowing for example a long shelf life, i.e. increasing the quality of storing beads and/or corresponding biosensor components comprising such beads after their manufacture. Alternatively or in addition thereto, the organic moiety may comprise one or more primers. Alternatively or in addition thereto, the organic moiety may comprise vectors. Alternatively or in addition thereto, the organic moiety may comprise a therapeutic or diagnostic or a binding agent or a carbohydrate or a proteinaceous material or a nucleotide sequence or a combination of any of these. The carbohydrate may be a sugar.

In other words, the beads are lyophilized beads, e.g. freeze dried beads. It is an advantage of embodiments of the present invention that the beads have a low degree of reactivity, thus allowing for example a long shelf life, i.e. improving storage properties of the beads and/or corresponding biosensor components comprising such beads after their manufacture.

As an optional feature, the organic moiety may comprise one or more sugars and/or the beads may comprise on or more salts. This is advantageous because sugars and salts provide for a fast dissolution of the beads.

As an optional feature the beads may have been monodispersed, resulting in substantial uniform size and/or shape.

As an optional feature, the lyophilized bead further comprises one or more proteins. This is advantageous because proteins stabilize the probes.

As an optional feature, if present, at least part of the organic moiety, e.g. one or more probes, primers, vectors etc. may be immobilized or retained on the one or more magnetic or magnetisable particles. This is advantageous because while the magnetic or magnetisable particles enable an easy deposition of the lyophilized beads at precise places of the sensor, the immobilization of the organic moiety, e.g. probes, primers, vectors, etc. on the magnetic particles permits the magnetic particles to serve as labels for the detection of interaction between the organic moiety, e.g. probes, primers, vectors, etc. and other objects such as analytes.

As another optional feature, the one or more probes may be synthetic or natural antibodies or fragments thereof. This is advantageous because it permits to analyze the presence of antigens in a sample fluid.

As another optional feature, the lyophilized bead may have a diameter of 10 to 300 µm. This is advantageous because this size range is compatible with the size of channels and containers (i.e. chambers) in microfluidic devices and with the size of wells in sensors such as e.g. biosensors. As yet another optional feature, all magnetic dipoles of the magnetic or magnetisable particles in the bead may be oriented along a same, predetermined orientation.

As yet another optional feature, the one or more sugars may be selected from the group consisting of mannitol, trehalose, sucrose and mixtures thereof. This is advantageous because those sugars enable to obtain stable and relatively amorphous spheroidal structure after freeze-drying. An advantage of mannitol is the fact that it is only weakly hygroscopic.

In another aspect of the invention, a process is provided for analyzing the presence of an analyte in a sample fluid. This process comprises providing the sample fluid, contacting the sample fluid with one or more beads, the one or more beads comprising one or more magnetic or magnetisable particles and an organic moiety (see above), the beads being lyophilized, and detecting the interaction between the sample fluid and the organic moiety (e.g. one or more probes comprised in the organic moiety). It is an advantage of embodiments of the present invention that an easy deposition of a well defined amount of reagent at precise places of a sensor or of a microfluidic system is enabled. As an optional feature, one or more probes may be comprised in the organic moiety and are therefore present in the beads.

As an optional feature, the one or more probes are retained or immobilized on the one or more magnetic or magnetizable particles and wherein the step of detecting comprises detecting interaction between the sample fluid and the one or more probes via the one or more magnetic or magnetizable particles. This is advantageous because while the magnetic or magnetisable particles enable an easy deposition of the lyophilized beads at precise places of the sensor, the immobilization of probes on the magnetic or magnetizable particles permits the magnetic or magnetizable particles to serve as labels for the detection of interaction between the probes and analytes. An additional label such as e.g. a fluorescent label is therefore not necessary.

As another optional feature, the step of detecting may comprise providing a sensor surface comprising analyte analogues immobilized thereon or therein, the analyte analogues being able to interact with the organic moiety, e.g. with one or more probes, contacting the liquid mixture with the sensor surface, detecting the presence of one or more magnetic or magnetizable particles on or in the sensor surface.

As another optional feature, the lyophilized beads may be located on or in the sensor surface. This is advantageous because it is simpler than to have them separated from the sensor and it permits faster analysis since the sample fluid is not required to travel the distance between a separated channel or container and the sensor.

As another optional feature, the step of contacting said sample fluid with one or more beads may be performed in a first container or channel separated from the sensor, the first container or channel comprising the lyophilized beads and being able to deliver the sample fluid to he sensor. This is advantageous because it ensures that no binding or mixing of the reagents occurs with the sensor surface prior to that the sample fluid contacts the reagent (enabling a pre-incubation step).

As another optional feature, the process comprises prior to the contacting, modifying the sample fluid by contacting the sample fluid with auxiliary beads in a second container or channel, the auxiliary beads comprising one or more modifying agents, the beads being lyophilized, and thereafter providing the modified sample fluid to the first container or channel. This is advantageous because it permits to tune the properties of the test fluid e.g. its viscosity, salt or detergent concentration among others. The auxiliary beads further may comprise one or more proteins.

As another optional feature, the analyte may be a drug of abuse. This is advantageous because drugs of abuse cause an important public health concern that would benefit from a fast testing method such as provided by embodiments of the present invention.

As yet another optional feature, the sample fluid may comprise saliva. This is advantageous because saliva is much more easily available than other sample fluids such as blood or urine.

In yet another aspect of the present invention, a sensor system is provided comprising one or more beads, the one or more beads comprising one or more magnetic or magnetisable particles and an organic moiety, the beads being lyophilized, the sensor system furthermore comprising a sensor surface. The sensor furthermore may comprise one or more chambers or channels, wherein the one or more beads are comprises in at least one of the chambers or channels. The organic moiety an therefore the bead further may comprise one or more probes.

In another aspect of the present invention, a method of manufacturing a sensor system is provided, the sensor system comprising one or more beads, the one or more beads comprising one or more magnetic or magnetisable particles and organic moiety, the beads being lyophilized, the method comprising providing a sensor surface, and providing a lyophilized beads in the sensor system. The lyophilized beads may be provided or applied to the sensor surface. The organic moiety and therefore the bead further may comprise one or more probes.

As an optional feature, the providing or applying may be performed by magnetic actuation. This is advantageous because magnetic actuation enables an accurate positioning and a soft, non-damaging manipulation of the beads.

In another aspect, the present invention relates to a system for manufacturing a lyophilized bead, the system comprising a container for containing a liquid mixture comprising the components of the lyophilized beads, a spraying or atomizing means with a spraying or atomizing nozzle for spraying the liquid mixture into a fluid medium and a functionally and/or geometrically adjusting means for adjusting the liquid mixture after leaving the spraying nozzle and prior to contacting the fluid medium. It is an advantage that prior to its lyophilization properties of the bead can be set, controlled and/or influenced. The functionally and/or geometrically adjusting means may be a magnetic or electromagnetic field generating means. The system may further comprise a detection means for visualizing the atomized or sprayed liquid mixture.

These and other aspects of the invention will be apparent from and elucidated with reference to the embodiment(s) described hereinafter. Particular and preferred aspects of the invention are set out in the accompanying independent and dependent claims. Features from the dependent claims may be combined with features of the independent claims and with features of other dependent claims as appropriate and not merely as explicitly set out in the claims.

The teachings of the present invention permit the design of improved methods and apparatus for bio-detection or bio-sensing.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference Figures quoted below refer to the attached drawings.

### BRIEF DESCRIPTION OF THE DRAWINGS

Fig. 1 is a schematic representation of a lyophilized bead according to embodiments of the present invention.
Fig. 2 is a schematic representation of a sensing system according to embodiments of the present invention.
Fig. 3 is a schematic representation of a system for an ink-jet mediated bead formation set-up according to an embodiment of the present invention.
Fig. 4 is a schematic representation of a system for micro-pipette mediated bead formation set-up according to an embodiment of the present invention.
Fig. 5 is a schematic representation of a system for analyzing the presence of an analyte according to an embodiment of the present invention.
Fig. 6 is a schematic representation of a system for manufacturing lyophilized beads according to an embodiment of the present invention.

In the different Figures, the same reference signs refer to the same or analogous elements.

### DETAILED DESCRIPTION OF THE EMBODIMENTS

The present invention will be described with respect to particular embodiments and with reference to certain drawings but the invention is not limited thereto but only by the claims. Any reference signs in the claims shall not be construed as limiting the scope. The drawings described are only schematic and are non-limiting. In the drawings, the size of some of the elements may be exaggerated and not drawn on scale for illustrative purposes.

Where the term "comprising" is used in the present description and claims, it does not exclude other elements or steps. Where an indefinite or definite article is used when referring to a singular noun e.g. "a" or "an", "the", this includes a plural of that noun unless something else is specifically stated.

Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Reference throughout this specification to "one embodiment" or "an embodiment" means that a particular feature, structure or characteristic described in connection with the embodiment is included in at least one embodiment of the present invention. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In the description provided herein, numerous specific details are set forth. However, it is understood that embodiments of the invention may be practised without these specific details. In other instances, well-known methods, structures and techniques have not been shown in detail in order not to obscure an understanding of this description.

The following terms or definitions are provided solely to aid in the understanding of the invention. The definitions should not be construed to have a scope less than understood by a person of ordinary skill in the art.

The term "sample", as used herein, relates to a composition which may comprise at least one analyte of interest. The sample is preferably fluid, also referred to as "sample fluid", e.g. an aqueous composition. The term "analyte", as used herein, refers to a substance whose presence, absence, or concentration is to be determined according to the present invention. Typical analytes may include, but are not limited to organic molecules, metabolites such as glucose or ethanol, proteins, peptides, nucleic acid segments, molecules such as pharmaceuticals, antibiotics or drugs, drugs of abuse, molecules with a regulatory effect in enzymatic processes such as promoters, activators, inhibitors, or cofactors, viruses, bacteria, cells, cell components, cell membranes, spores, DNA, RNA, micro-organisms and fragments and products thereof, or any substance for which attachment sites, binding members or receptors (such as antibodies) can be developed.

The term "label", as used herein, refers to a molecule or material capable of generating a detectable signal or capable of binding to another molecule or forming a complex which generates a detectable signal. Suitable labels for use in different detection systems and methods of the present invention are numerous and extensively described in the art. These may be optical labels (e.g. luminescent molecules (e.g. fluorescent agents, phosphorescent agents, chemiluminescent agents, bioluminescent agents and the like), colored molecules, molecules producing colors upon reaction), radioactive labels, magnetic and/or electric labels, enzymes, specifically bondable ligands, microbubbles detectable by sonic resonance and the like. Labels can be direct labels, which can be detected by a sensor. Alternatively, labels can be indirect labels, which become detectable after a subsequent development process. Typically, the label used in the methods of the present invention is an analyte-specific label, i.e. capable of binding specifically to the analyte. Nevertheless, it is also envisaged that where the analyte is present in a purified form, it is sufficient that the label binds to the target.

The term "analyte analogue", as used herein, refers to a substance that can associate with a probe or capture probe. The analyte analogue is used in competitive assays where the analyte is determined based on competition with the analyte analogue, e.g. in the competitive binding to a probe or capture probe. The term "probe" relates in the present invention to a binding molecule that specifically binds an analyte. Probes envisaged within the context of the present invention include biologically-active moieties such as but not limited to whole anti-bodies, antibody fragments such as Fab' fragments, single chain Fv, single variable domains, VHH, heavy chain antibodies, peptides, epitopes, membrane receptors or any type of receptor or a portion thereof, substrate-trapping enzyme mutants, whole antigenic molecules (haptens) or antigenic fragments, oligopeptides, oligonucleotides, mimitopes, nucleic acids and/or mixture thereof, capable of selectively binding to a potential analyte. Antibodies can be raised to non-proteinaceous compounds as well as to proteins or peptides. Probes are typically members of immunoreactive or affinity reactive members of binding-pairs. The nature of the probe will be determined by the nature of the analyte to be detected. Most commonly, the probe is developed based on a specific interaction with the analyte such as, but not limited to, antigen-antibody binding, complementary nucleotide sequences, carbohydrate-lectin, complementary peptide sequences, ligand-receptor, coenzyme, enzyme inhibitors-enzyme, etc. In the present invention, the function of a probe is specifically interact with an analyte to permit its detection. Therefore, probes may be labeled or may be directly or indirectly detectable. The probe can be an anti-analyte antibody if, for instance, the analyte is a protein. Alternatively, the probe can be a complementary oligonucleotide sequence if, for instance, the analyte is a nucleotide sequence. The term "capture probe" as used herein, refers to probes for immobilizing analytes and/or labeled analytes on the sensor (e.g. its detection surface) via recognition or binding events.

The term "sensor" as used herein, refers to a device allowing qualitative and/or quantitative detection of an analyte in a sample fluid. If the analyte is of biological nature or if the sensor relies on biological entities for the detection, (e.g. antibodies capture probes) the sensor will sometimes be referred as a "biosensor". The "sensor" as used herein comprises a sensing substrate. If non-porous, the sensing substrate usually operates its sensing through a sensing surface that will either capture analytes or exchange an analyte analogue immobilized thereon for an analyte present in the sample fluid. In this case, the sample fluid is "flowing over" the sensing surface. If the sensing substrate is porous, the sensing substrate usually operates its sensing through its whole thickness and the entities used for the detection (e.g. the antibodies capture probes) are distributed throughout the thickness of the sensing substrate. In this case, the sample fluid is "flowing through" the sensing substrate. In this case, the sample fluid is "flowing through" the sensing substrate.

The term "proteinaceous material" refers to a material comprising amino-acids such as but not limited to a material comprising peptides, proteins, glycoproteins and the likes.

In the different aspects and embodiments according to the present invention, the fluid mixture may be a suspension or a slurry. In the fluid mixture the different components need not be in solution.

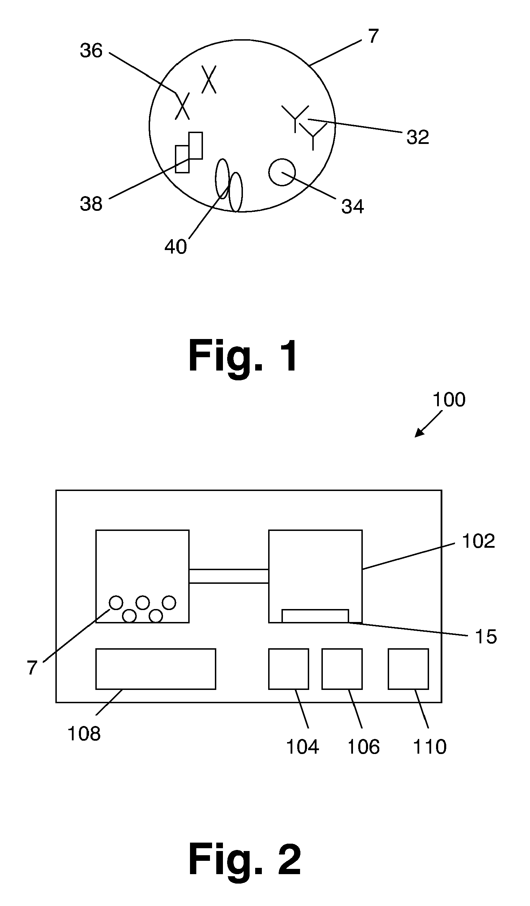

In a first aspect, the present invention relates to a bead or plurality of beads, e.g. for use in biosensing applications. A bead according to the present invention comprises one or more magnetic or magnetisable particles and an organic moiety. The organic moiety may be a matrix. The organic moiety may comprise a binding material to bind specifically to predetermined biological or biotechnological components or other components such as a surfactant. The organic moiety may comprise one or more probes. Alternatively or in addition thereto, the organic moiety may comprise one or more primers. The primers thereby are pieces of DNA which may be used in polymer chain reactions (PCR) to amplify samples of DNA. The primers may bind to the complementary pieces of a DNA sequence. Alternatively or in addition thereto, the organic moiety may comprise a vector. A vector may guide an attached entity to somewhere else, e.g. it may guide an entity into a cell through the cell membrane. Alternatively or in addition thereto, the organic moiety may comprise a therapeutic or diagnostic or a binding agent or a carbohydrate or a proteinaceous material or a nucleotide sequence or a combination of any of these. The carbohydrate may be a sugar. The organic moiety thusmay comprise one or more sugars and/or polymers. The beads may comprise one or more salts. The bead further is a lyophilized bead, i.e. dried by freezing for example in a vacuum. The lyophilized bead may have a variety of morphologies and shapes. Lyophilization or freeze-drying results in a lowered reactivity of the beads, thus resulting in a longer shelf life, i.e. improved storing properties. Such beads may be stored as beads in a recipient or may be stored incorporated in corresponding sensor devices or parts thereof. Exemplary shapes include spherical, near spherical, elliptical or round structures. According to the present invention, the bead may be droplet shaped. Exemplary morphologies include smooth or roughened surfaces. The lyophilized beads may have for instance a diameter of 10 to 300 µm. The size dispersity of the lyophilized beads is preferably substantially monodisperse, i.e. within a few percents, preferably less than 2%, even more preferably less than 1%. The particles may be made by monodispersion, resulting in particles having a substantially uniform shape and size. The size of the beads may be selected as to provide predetermined bio reagents, such as e.g. probes, for a given biotechnological application. By way of example, an exemplary bead is illustrated in Fig. 1, the bead 7 being lyophilized. The bead 7 comprises one ore more magnetic or magnetisable particles 34 and an organic moiety. Optionally, the organic moiety comprises one or more probes 32, one or more sugars 38, one or more proteins 40 or one or more polymers (not depicted). Optionally, the bead 7 comprises one or more salts 36.

The one or more probes 32 typically may be binding molecules that specifically bind an analyte. Optional features and examples of such probes are as set out above. Magnetic or magnetisable particles 34 may be, but are not limited to, metal or magnetic beads or nanoparticles. The magnetic or magnetisable particle 34 may include any suitable form of one or more magnetic particles or magnetisable particles 34 e.g. magnetic, diamagnetic, paramagnetic, superparamagnetic, ferromagnetic that is any form of magnetism which generates a magnetic moment in a magnetic field, either permanently or temporarily. Preferably, superparamagnetic particles are used since their use avoids possible aggregation problems that can occurs when permanent magnets are used. Examples of suitable magnetic particle material are e.g. Fe₃O₄ beads. The size of the magnetic particle is not critical in most embodiments but preferably have a longest dimension in the range 5 nm to 5000 nm, more preferably 30 nm to 1000 nm, most preferably between 80 nm and 500 nm. Unless specified otherwise, the terms "magnetic or magnetisable particles" refer the particles, molecules or material as such, not covalently linked to a probe.

In some preferred embodiments, the organic moiety, e.g. matrix component, comprises one or more sugars 38. Such sugars 38 may e.g. be selected from the group consisting of polyols, monosaccharides, disaccharides, oligosaccharides and polysacharides. For instance, they can be selected from sucrose, glucose, trehalose, melezitose, dextran or mannitol among others. Preferably, the one or more sugars 38 that may be part of the matrix component may be selected from the group consisting of polyols, disaccharides and oligosaccharides. In some embodiments, trehalose, mannitol or mixture thereof are used. One role of the one or more sugars may be to form a water-soluble and relatively amorphous matrix component. An amorphous matrix component permits a faster dissolution than a crystalline matrix component, thus assisting in beads that are preferred if fast availability of the bio-reagents is preferred or required. It is an advantage of such lyophilized bead that faster mixing occurs between the sample fluid and the probes than would occur when the probes would be delivered as fluid.

As indicated above, optionally one or more salts 36 may be part of the composition of the lyophilized bead. The one or more salts may be selected from the group comprising KCl and NaCl. Optionally, furthermore one or more proteins 40 may be comprised in the composition of the lyophilized bead. Examples of proteins 40 that can be comprised into the composition of the lyophilized bead comprise but are not limited to Bovin Serum Albumin (BSA), gelatine, and collagen among others. A role of the one or more proteins 40 is to stabilize the bead. The proteins will stabilize the probes and will have a positive effect on the shelf-life of the lyophilized beads.

The lyophilized bead according to the present embodiment comprises magnetic or magnetizable particles and can therefore be manipulated and positioned, e.g. on a sensor, by using magnetic actuation. It thus is an advantage of beads according to the present invention that an easy deposition of a well defined amount of reagent at precise places of a sensor or of a microfluidic system.

As an optional feature, the magnetic or magnetisable particles comprise the one or more probes immobilized thereon. This optional feature permits the magnetic or magnetisable particles to play a double role: 1) they serve to ease the manipulation and the positioning of the lyophilized beads and 2) they serve to label the one or more probes. The magnetic or magnetisable particle may be attached to the probe(s) preferably through covalent binding but other types of binding such as physisorption are also possible. For the purpose of binding magnetic or magnetisable particles to probes, amino-derivatized, carboxylic acid-derivatized and epoxy-derivatized magnetic particles (among others) are commercially available and can be prepared by methods extensively described in the literature.

In a second aspect, the present invention relates to a method for manufacturing a lyophilized bead. The lyophilized bead 7 can be manufactured by providing a liquid mixture comprising magnetic or magnetisable particles 34 and an organic moiety , creating the bead from the liquid mixture by atomizing or spraying the liquid mixture into a fluid medium and freezing it and finally freeze-drying the bead. The organic moiety may be a matrix. The organic moiety may comprise a binding material to bind specifically to predetermined biological or biotechnological components. The organic moiety may comprise one or more probes. Alternatively or in addition thereto, the organic moiety may comprise one or more primers. The primers thereby are pieces of DNA which may be used in polymer chain reactions (PCR) to amplify samples of DNA. The primers may bind to the complementary pieces of a DNA sequence. Alternatively or in addition thereto, the organic moiety may comprise a vector. A vector may guide an attached entity to somewhere else, e.g. it may guide an entity into a cell through the cell membrane. Alternatively or in addition thereto, the organic moiety may comprise a therapeutic or diagnostic or a binding agent or a carbohydrate or a proteinaceous material or a nucleotide sequence or a combination of any of these. The organic moiety may for example comprise sugars and/or polymers, and optionally comprises proteins. Droplets thereby can be made by dividing the liquid mixture in small volumes. As will be illustrated below, such dividing may be based on inkjet technology or using any other suitable droplet forming system. The liquid mixture is usually of aqueous base but may comprise also other water miscible co-solvents such as ethanol or isopropanol among others. The freezing step, wherein a volume, e.g. droplet, of the liquid mixture is provided to the fluid medium, is performed by spraying the liquid mixture in the fluid medium. Spraying the liquid mixture may comprise adding the droplets to a fluid medium having a temperature substantially lower than the freezing temperature of the liquid mixture from which the droplets are made. The droplets may be of a predetermined size and/or shape. Adding droplets may be performed by jetting droplets in a droplet stream into the fluid medium. Furthermore, adding a droplet to the fluid medium may comprise monodisperse droplet formation and jetting. The freezing temperature of the liquid mixture must be higher than the temperature of the liquid medium, preferably at least 50°C higher, more preferably at least 75 °C higher and most preferably at least 100 °C higher. This is to ensure a fast freezing of the droplets and to avoid the formation of large crystals in the beads. The freezing temperature of the liquid composition will usually be 0°C or below, function of the concentration of its constituents. The liquid mixture may be provided by mixing the different components e.g. in a recipient. There is a large variety of possibilities to create the liquid composition and the person skilled in the art can chose the method that best suites his needs. For instance, the various components can be added either one at a time or two or more at a time, into a container which may contain a stirring mean, that can be manually shaken or stirred or within which a mixing can be performed by any means such as but not limited to the use of a standard propeller, a disperser, a sonicator, a roller mill, a vortex or a shaker among others. Cooling the liquid composition (e.g. if heat sensible material is present in the liquid composition) or warming up the liquid composition (e.g. to ensure solubility of one or more of the components) while mixing may be useful. The liquid composition may by way of example, the invention not being limited thereto, comprise, in addition to the magnetic or magnetisable particles 34, as part of the organic moiety, one or more sugars 38, one or more probes 32, one or more proteins 40, one or more polymeric excipients such as but not limited to polymers such as polyethyleneglycol (PEG), polyvinyl pyrrolidone (PVP) or starch among others, detergents such as but not limited to Tween-20 among others, etc... The liquid composition can further comprise salts such as but not limited to KCI and NaCI and/or inorganic ions such as but not limited to borate, phosphate or carbonate among others. The one or more sugars 38 and/or salts 36 can be present in largely varying proportions. For instance they can be present in proportions between 0.5%wt to 80%wt, preferably, they are present in proportions between 3-10wt%. If present, the one or more proteins can be present in largely varying concentration. For instance they can be present in concentration between 0.5 mg/ml and 20 mg/ml, preferably they are present in concentration between 1 mg/ml and 10 mg/ml. The one or more excipients can be present in largely varying proportions. For instance they can be present in proportions between 0.5%wt to 80%wt, preferably, they are present in proportions between 0.5-10wt%.The one or more probes can be present in largely varying amounts, for instance in the range 1 to 30 µg/mg. The magnetic or magnetisable particles can be present in largely varying proportions. For instance 0.1-1 wt%.

Once provided with a liquid mixture as described above, the method also may comprise a step of dividing this liquid mixture into droplets. This can be performed by various dividing means such as the use of a spotting device, a pipette, a micro-pipette or a pump. The delivery of the drop can be activated by any means known by the person skilled in the art such as but not limited to piezo-electric means, piston mediated means and the likes. For instance, an ink-jet pump or a microdrop pipette can be used. Preferably, ink-jet technology (i.e. an ink-jet pump) is used in order to achieve small bead sizes and a relatively narrow distribution of bead sizes. A narrow distribution of the lyophilized bead sizes is useful because it permits to correlate the number of lyophilized beads with the number of probes and/or the number of magnetic or magnetisable particles. It is indeed preferable in some embodiments of the present invention that each bead contains a known amount of probes and/or magnetic or magnetisable particles. The size of the droplets can be tuned by various means such as varying the size of the pump or pipette nozzle or by varying the pulse duration of dispersing the liquid mixture (e.g. the pulse duration applied to the piezo of an ink-jet pump).

The method furthermore comprises spraying the liquid mixture in the fluid medium having a temperature lower than the freezing temperature of the liquid composition. Spraying the liquid mixture may comprise adding the droplets to a fluid medium having a temperature substantially lower than the freezing temperature of the liquid mixture from which the droplets are made. The droplets may be of a predetermined size and/or shape. Adding droplets may be performed by jetting droplets in a droplet stream into the fluid medium. Furthermore, adding a droplet to the fluid medium may comprise monodisperse droplet formation and jetting. Droplet formation may be performed in any suitable way, as set out above. Spraying the liquid mixture to the fluid medium may be performed simultaneously with formation of the droplets, i.e. by simply dropping the liquid composition into the liquid medium. The frequency at which the drops should be added to the liquid medium should not be too high in order to avoid aggregation of newly formed beads on previously formed beads. This frequency should preferably be chosen below 50 Hz, preferably below 40 Hz, most preferably around 30 Hz or lower. The height of the nozzle of the dividing means to the surface of the liquid medium is preferably sufficiently large to avoid freezing of the liquid composition at or in the nozzle. For this purpose, a distance between 2 and 10 cm can for instance be chosen in an exemplary experimental setup. The fluid medium should in any case be colder than the freezing temperature of the liquid composition. Preferably the freezing medium is at a temperature of at least 50°C colder, more preferably at least 75 °C colder and most preferably at least 100 °C colder than the freezing temperature of the liquid composition. For instance, the fluid medium can be chosen as having a temperature below - 75°C or below -100 °C. Preferably, the fluid medium is a cryogenic liquid, i.e. a liquid at a temperature below -150°C. In an exemplary embodiment, the fluid medium is nitrogen or a freon. A cryogenic fluid medium is usually preferred to avoid phase separation of the particles and the liquid in the lyophilized beads. Another step of the method comprises freeze-drying (lyophilizing) the obtained bead(s). Freeze drying can be performed in a standard freeze drier. In some embodiments, the degree of vacuum is controlled in order to avoid expansion of the beads. Additionally, a dry inert gas (e.g. nitrogen) flow in the chamber can be provided to carry moisture away from the bead material. The lyophilization of the beads ensures an improved shelf-life of the probes, lessen the tendency of the magnetic particles to aggregate and ensure a good dispersion of the probes and magnetic particles after contacting with the sample fluid.

In one embodiment, during their formation, the beads may be functionally or geometrically adjusted during the spraying. During the spraying, i.e. after the liquid mixture has left a spraying or jetting nozzle, the liquid mixture falls over a significant distance before the droplets enter the fluid medium during which the liquid mixture may be functionally or geometrically adjusted. The distance over which the liquid mixture falls may be in the range of a couple of centimeters, i.e. between 3 cm and 20cm. If for example droplets are sprayed from the nozzle, the droplets may be functionally or geometrically adjusted. In one example, the present invention not being limited thereto, the speed of the liquid mixture after leaving the nozzle is between 5 m/s and 10 m/s, thus providing a couple of milliseconds for performing such functional or geometrical adjustment.

The functional or geometrical adjustment may e.g. be performed by applying a magnetic and/or electromagnetic field to the liquid mixture, e.g. droplets thus influencing the liquid mixture by influencing the magnetic or magnetisable particles. Such a magnetic and/or electromagnetic field may e.g. be of the order of 0.1 to 1 Tesla or more. Such magnetic and/or electromagnetic fields may be applied using an (electro)magnetic field generating means, such as e.g. a permanent magnetic or an electromagnet. The generated magnetic and/or electromagnetic field may be an AC or DC field.

One example of functional or geometrical adjustment may be orientation of a magnetic multipole, e.g. dipoles, present in the form of magnetic or magnetisable particles. Such orientation may be based on electrorheological and/or magnetorheological effects whereby e.g. chain formation occurs. The magnetic multipoles obtained in this way may be oriented in a predetermined direction and may be lyophilized in this way, resulting in a fixed orientation of the magnetic or magnetisable particles in the lyophilized beads. In this way a cumulative magnetic multipole, e.g. dipole, moment is obtained for the lyophilized beads. The latter may e.g. be advantageous for selection, positioning or orienting of lyophilized beads or for particular applications of the beads.

Another example of a functional or geometrical adjustment may comprise adjusting a speed and/or geometrical property such as e.g. shape of the liquid mixture, e.g. droplet. Such a speed and/or shape adjustment may also be performed by inducing a magnetic force on the particles, e.g. as described above. For example shape of magnetic liquids, magneto-rheological liquids or magnetic compound liquids may be influenced by providing them in alternating magnetic fields. The shape furthermore may reflect the cluster structure of particles being present in the liquid mixture. In one example, a variable magnetic field may be generated by an array of planar coils in combination with a permanent magnet field, whereby the speed and/or of the sprayed liquid mixture is controlled by modulating the magnetic field applied to the particles.

Still another example of a functional or geometrical adjustment is a change in position of the liquid mixture, e.g. of droplets. Such a change in position of the liquid mixture may be a deflection of the liquid mixture by applying a magnetic or electromagnetic field. The magnetic or magnetisable particles in the liquid mixture, e.g. in the droplets, thus may be used for positioning. Furthermore, positioning may be used for selecting and/or classifying droplets as for a given amount of magnetic or magnetisable particles being present in a volume or droplet of liquid mixture and for a given magnetic deflecting means, the liquid mixture will deflect over a given distance. In other words, the distance over which deflection has occurred is a measure of the amount of magnetic or magnetisable particles being present in a volume or droplet of liquid mixture.

Another optional feature of the present invention is to provide a detection system for detecting the presence of particles and optionally providing feedback to the manufacturing system. In this way, if something goes wrong in the manufacturing process, feedback can be provided. Such detection may e.g. be imaging of the droplets. Suitable imaging systems that can be used may e.g. be as known for droplet formation in ink-based printing. Measuring features like droplet shape, size and volume of a droplet can e.g. include a visual inspection system like for example a VisionJet Optica system commercially available from Xennia Technologies Ltd. For determining these features, e.g. laser-based imaging systems like stroboscopic imaging may be used.

The sprayed liquid mixture, e.g. droplets, may be provided through a channel through electromagnetic coils. In a particular example, the particle size distribution may be determined. The latter may e.g. be performed using Tyndall spectra. Using an irradiation beam passing through a monodispersed colloid wherein the radius of the dispersed particles is roughly comparable in magnitude with the wavelength of the irradiation, Higher order Tyndall spectra may be obtained by scattering of the irradiation. Based on such Tyndall spectra, droplet sizes may be determined. Alternatively, instead of optical detection using imaging, presence of particles and thus good operation of the system could also be determined using a magnetic sensor, such as e.g. a Hall sensor or magnetoresistive sensor. The corresponding feedback system may comprise a processor for analyzing the obtained detection results and providing a control signal to a controller in the manufacturing system based on predetermined criteria like amount of liquid mixture to be sprayed in a given time duration. If the manufacturing system starts working incorrectly, an appropriate action may be carried out such as an alarm can be sounded or the system can for example be shut down and maintained.

Furthermore, such detecting steps may also comprise applying techniques for detecting or measuring magnetic properties, such as e.g. the presence of magnetic material in the droplet, a magnetic moment being present, etc. By way of example, the invention not being limited thereto, X-ray micro-tomography techniques or micro-structural analysis techniques may be used.

In a third aspect, the present invention relates to a process for analyzing the presence of an analyte in a sample fluid. This process comprises the steps of providing the sample fluid, contacting the sample fluid with one or more lyophilized beads as described in the first aspect and detecting the interaction between the sample fluid and the one or more probes introduced via the lyophilized beads. Contacting the sample fluid with the one or more lyophilized beads preferably comprises forming a mixture of the components of the lyophilized beads and the sample fluid. The sample fluid can be from industrial or natural origin. Examples of sample fluids suitable for performing the method of this invention may be, but are not limited to, body fluids such as sputum, blood, urine, saliva, faeces or plasma from any animal, including mammals (especially human beings), birds and fishes. Other non-limiting examples include fluids containing biological material from plants, nematodes, bacteria and the like. For a suitable performance of embodiments according to the analyzing process of this invention it is preferred that the sample is present in a substantially fluid, preferably liquid form, for instance in solution in a suitable dissolution medium. The sample fluid can be used as such or can be modified (e.g. concentrated, diluted, made more or less viscous, introduction of a buffer, etc...). The contact between the sample fluid and the one or more lyophilized beads can occur either on the sensor surface or in a first container or channel separated from the sensor surface and being able to receive the sample fluid and to deliver the sample fluid to the sensor. As an optional feature, auxiliary lyophilized beads, for example comprising one or more modifying agents and optionally one or more sugars and/or salts and one or more proteins can be comprised in a second container or channel being able to receive the sample fluid and to deliver the modified sample fluid to the first container or channel. The one or more modifying agents can comprise for instance viscosity modifiers, salts, buffers, detergents among others. As an optional feature, the auxiliary lyophilized beads comprises a modifying agent and/or a second type of probes directed to a second analyte suspected in the sample. Additional auxiliary lyophilized beads for additional analytes are of course encompassed by these embodiments of the present invention. Similarly, more than one type of probes could be present in the one or more probes of a lyophilized bead according to embodiments of the present invention.

The occurrence of a contact on the sensor surface may be preferred because the distance between the lyophilized beads and the analyte analogues or the capture probes immobilized on the sensor is in this case minimal, which permits a relatively higher speed of analysis than when the lyophilized beads are in a container or channel separated from the sensor. Alternatively, the occurrence of a contact in a container or channel separated from the sensor may be preferred because it reduces the risk of interaction between the lyophilized beads and the sensor prior to a contact with the sample fluid. The deposition of the lyophilized beads on the sensor (or in the sensor, if the sensor has pores larger than the lyophilized particles), can be performed accurately by magnetic actuation. This is made possible by the fact that the lyophilized beads of the present invention comprises magnetic or magnetisable particles. The magnetic force that can be used for the manipulation of the lyophilized beads, e.g. for the deposition of the lyophilized beads on the sensor, in a channel or in a container, can be applied by any suitable magnetization means such as e.g. coils and/or magnetic materials such as but not limited to magnetic coils in which the current is controlled, an electromagnet with a magnetic core having a small tip or a permanent magnet e.g. with a small tip that is mechanically moved (e.g. retracted) or used with a mechanical barrier (e.g. the well of a fluidic channel) between the permanent magnet and the lyophilized beads. Once the lyophilized beads are contacted with the sample fluid, the lyophilized beads dissolve and liberate their content. Thereafter or simultaneously therewith, the sample fluid is contacted with the sensor surface and wets it (or penetrates its surface if the sensor is porous).

The detection of the interaction of the sample fluid with the one or more probes comprises the detection of the analyte via detection of the probes. The probes (e.g. the labeled antibodies) and the sensor are both exposed to the analyte and the analyte influences the binding of the probes to the sensor surface. Depending on the type of assay being performed, an analyte labeled with e.g. a magnetic or magnetisable particle (via a probe) either bind to immobilized capture probes (sandwich assay), or compete with analyte analogues to bind to capture probes (competitive assay). After removal of excess (unbound) labeled probes (which in some embodiments is equivalent with the removal of the magnetic or magnetisable particles), the amount of bound labeled probes (e.g. labeled with magnetic particles) can be measured. Thus, binding assays may typically involve adherence of magnetically labeled molecules to the sensor in numbers that reflect the concentration or presence of the analyte molecule.

A large number of variations on binding assay methodologies have been described and are all within the scope of the present invention. Detection of a magnetic or magnetisable particle when used as a label is generally done by application of an electric, or magnetic, or electromagnetic field and using a magnetic or non-magnetic, e.g. optical or acoustic sensor. Examples of embodiments for the detection of a magnetic or magnetisable particle are given in patent application WO2005/11666 and in references cited therein. Acoustic and/or sonic detection of labels may also be used. In some embodiments, the magnetic particles are only presents in the lyophilized beads to enable their manipulation via magnetic means, i.e. magnetic actuation and do not serve as labels. In those embodiments, the detection of the probes on or in the sensor will be adapted to the type of label linked to the probes. Also, the various types of binding and releasing assays may use magnetic particles that comprise optical properties such as e.g. fluorescent, chromogenic, scattering, absorbing, refracting, reflecting, SERRS-active or (bio)chemiluminescent labels, molecular beacons, radioactive labels, or enzymatic labels. Optically active labels typically may emit light detectable by a detector, e.g. in the visual, infrared or ultraviolet wavelength region. Nevertheless, the invention is not limited thereto and optical labels, in the present application, may refer to labels emitting in any suitable and detectable wavelength region of the electromagnetic spectrum.

In a particular embodiment, the detection of the interaction between the sample fluid (e.g. the analyte within) and the one or more probes comprises providing a sensor comprising analyte analogues immobilized thereon or therein, the analyte analogues being able to interact with the one or more probes, contacting the mixture of sample liquid and the contents of the lyophilized beads with the sensor, and detecting the presence of one or more magnetic or magnetisable particles on or in the sensor.

In a fourth aspect, the present invention relates to a sensor system comprising one or more lyophilized beads, the one or more lyophilized beads comprising one or more probes and one or more magnetic or magnetisable particles, i.e. beads as described in the first aspect, comprising the same features and advantages. The sensor system furthermore may comprise a sensor surface, whereon detection may be performed. The sensor surface may be adapted for different types of detection, as described above in the third aspect. Furthermore, the sensor surface may comprise particles adapted for interacting, e.g. selectively interacting with analytes or components bound thereto. The sensor system may comprise one or more containers, e.g. a container wherein mixing occurs and a container wherein the sensing or detecting occurs. The mixing and detecting container may be one and the same container. The sensor system preferably is adapted for detecting an optical or magnetic signal representative of the presence and/or quantity of the analytes in the sample. The sensor system therefore may comprise an excitation means and detector means. Furthermore, the sensor system may comprise a magnetic actuating means for positioning during manufacturing or use, the lyophilized beads and/or the magnetic particles e.g. coupled to the probes. Such a sensor can be easily processed, stored and operated at relatively high temperature without suffering from deterioration. The sensor system furthermore may comprise a processing unit for processing the obtained detection/sensing results. Whereas the different components such as excitation means, detector means, actuating means and processing unit have been illustrated as parts of one sensor system, these may also be positioned in separate parts, such that the sensor system may consist of a disposable cartridge system and a cartridge reader that typically is used repeatedly. By way of example, a schematic representation of such a sensor system 100 comprising lyophilized beads 7 and a sensor surface 15 is shown in Fig. 2. As described above, the sensor system 100 furthermore may comprise one or more containers 102, an excitation means 104, a detection means 106, an actuating means 108, a processing unit 110 and other typical components of sensor systems as known by the person skilled in the art. The sensor system can for instance be a microfluidic system.

In yet another aspect, the present invention relates to a method for the manufacture of such a sensor system 100. The method comprises the steps of providing a sensor surface and providing lyophilized beads in the sensor system 100. The lyophilized beads thereby comprise one or more probes and one or more magnetic or magnetisable particles. Further optional components also may be present. The lyophilized thus may be as described in the first aspect, comprising the same features and advantages. As an optional feature, the manufacturing method may comprise actuating the lyophilized beads to provide them on the appropriate place in the sensor system, e.g. but not limited to the sensor surface. The magnetic actuation that can be used for the manipulation of the lyophilized beads, e.g. for the deposition of the lyophilized beads on the sensor, in a channel or in a container, can be applied by coils and/or magnetic materials such as but not limited to magnetic coils in which the current is controlled, an electromagnet with a magnetic core having a small tip or a permanent magnet with a small tip that is mechanically moved (e.g. retracted).

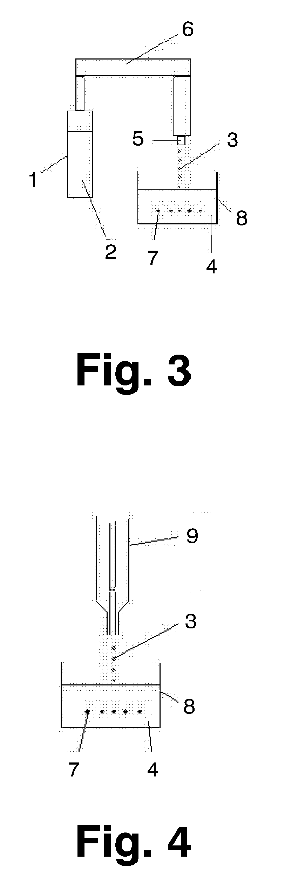

By way of example, the present invention not limited thereto, some exemplary setups are shown in Fig. 3 to Fig. 5, the corresponding embodiments and aspects of the present invention not being limited thereto.

In Fig. 3, a system for an ink-jet mediated bead formation method according to an embodiment of the present invention is disclosed. Fig. 3 shows a container 1 containing a liquid mixture 2 comprising the components of the lyophilized beads. The liquid mixture 2 creates an underpressure in the system. The container 1 is connected to an ink-jet pump 6 comprising a nozzle 5. The ink-jet pump 6 sucks liquid mixture 2 from container 1 and divides the liquid mixture 2 into droplets 3 at the level of nozzle 5. Droplets 3 are added into liquid medium 4 contained in vessel 8 and form beads 7. The beads are then quickly transferred to a freeze drying apparatus (not shown) where they will be lyophilized.

In Fig. 4, a system for a micro-pipette mediated bead formation method according to an embodiment of the present invention is disclosed. Fig. 4 shows a micro-pipette 9 dividing the liquid composition 2 into droplets 3. Droplets 3 are added into liquid medium 4 contained in vessel 8 and form beads 7. The beads are then quickly transferred to a freeze drying apparatus (not shown) where they will be lyophilized. Attached on the pipette is an underpressure-regulation apparatus (not shown).

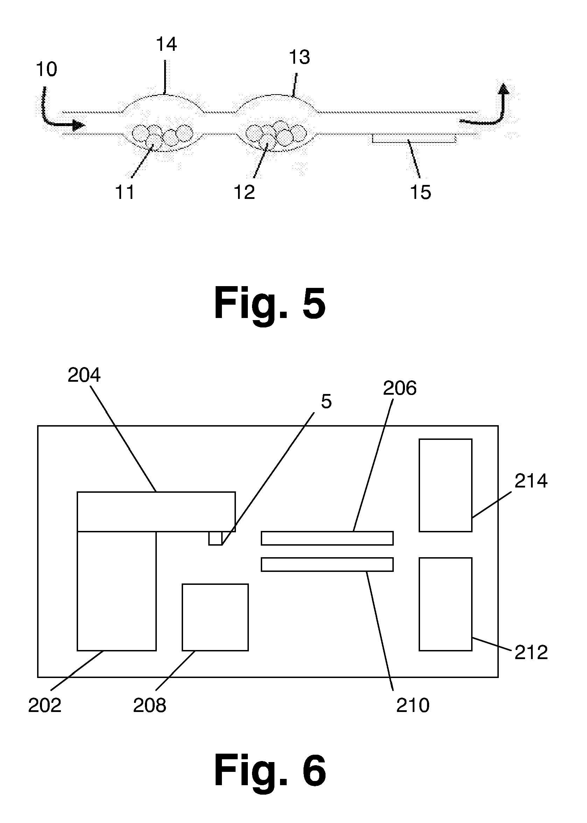

In Fig. 5, a schematic representation of a process for analyzing the presence of an analyte according to an embodiment of the present invention is disclosed. Fig. 5 3 shows a system composed of a first chamber 13 comprising lyophilized beads 7, a second chamber 14 in communication with first chamber 13, such that fluid can flow between the first chamber 13 and second chamber 14 and comprising auxiliary lyophilized beads 11 and a sensor surface 15 in fluid communication with the first chamber 13. A sample fluid 10 is shown to be introduce in the system and will contact the auxiliary beads 11 in the second chamber 14. The sample fluid 10 will be modified by this contact and will subsequently enter the first chamber 13 where it will contact the lyophilized beads 7. The so formed liquid mixture will then contact the sensor surface 15 where the analyte detection will be performed.
Furthermore some explicit examples of lyophilized beads and their manufacturing are described to illustrate features and advantages of the first and second aspect, embodiments of the first and second aspect not being limited thereto.

### Example 1: magneticle particle functionalization

An aqueous solution comprising 0.05 wt% of streptavidin coated magnetic particles (diameter 200 nm, Ademtech, item code 03122) and 90 µg/ml of a biotinilated antibody (anti-morphine monoclonal mouse IgG) was stirred at 1000 rpm for 15 min. A stock of biotin solution (1 mg / ml) was diluted 10000 times and this diluted solution was added to magnetic particle containing solution in a ratio 1/10 (v/v). The magnetic particles where thereafter washed with a PBS buffer by using a magnetic separator. The beads are then stored in an Ademtech storage buffer.

### Example 2: liquid composition formation

50 mg of bovine serum albumine (BSA MW ~66 kDa), 0.5 g D-mannitol (MW=182.17) and 200 µl of an aqueous suspension comprising coated magnetic particles as obtained in example 1 (magnetic particle content = 0.55wt%) were individually added to a stirred solution of water to get a final weight of 10 g. Stirring was accomplished with a stir bar and stir plate. Each component was allowed to dissolve completely before the next component was added. The solution was used fresh.

### Example 3: Lyophilized bead formation

Bead formation was conducted using a Microdrop MD-K-140H inkjet pump with a 50 µm nozzle, without restriction. The system was loaded with the liquid composition. The nozzle of the pump was placed at approximately 5 cm above the cup filled with liquid nitrogen. The pulse applied on the piezo of the pump, was optimized: the pulsewidth was 30 µs (which it is the ground tone) and the voltage was about 70 V. The droplets were dispensed into liquefied nitrogen contained in a quartz vessel. The quartz vessel was placed in a second, larger vessel filled with ice to prevent rapid evaporation of liquid N₂. The printing frequency has been chosen very low (about 30 Hz), to prevent the printed spheres from sticking to each other in the liquid nitrogen. The freezing of the droplets occurred very rapidly since the droplets were very small (diameter on the order of 200 µm).

At the end of the bead forming process, the beads, in liquid N₂, were transferred to a -80°C freezer. Samples were kept in a -80°C freezer before freeze drying. Samples were kept in the freeze drier (Christ epsilon 2-6) at a shelf temperature of -10C for an hour at atmospheric pressure, after which the pressure was reduced to 1 mbar over a period of one hour. Subsequently the samples were left at this pressure for 20 hours. At the end of the freeze-drying process the freeze-drier was filled with nitrogen gas. The samples were removed and stored.

While the invention has been illustrated and described in detail in the drawings and foregoing description, such illustration and description are to be considered illustrative or exemplary and not restrictive; the invention is not limited to the enclosed embodiments. Other variations to the disclosed embodiments can be understood and effected by those skilled in the art in practising the claimed invention, from a study of the drawings, the disclosure, and the appended claims.

For example, whereas a method for manufacturing lyophilized beads has been described, the present invention also relates to a system for manufacturing lyophilized beads. Such a system 200 comprises a container 202 for containing a liquid mixture comprising the components of the lyophilized beads, a spraying or atomizing means 204 with a spraying nozzle 5 for spraying or atomizing the liquid mixture 2 into a fluid medium 4 and a functionally and/or geometrically adjusting means 206 for adjusting the liquid mixture after leaving the spraying nozzle and prior to contacting the fluid medium 4. The fluid medium may be contained in a fluid medium container 208. The functionally and/or geometrically adjusting means 206 may for example be a magnetic or electromagnetic field generating means. Such a magnetic or electromagnetic field generating means may for example be adapted for generating a magnetic field having a magnitude of 0.1-1 Tesla or higher. The magnetic or electromagnetic field generating means 206 preferably is positioned near the path of the liquid mixture between the spraying nozzle and the fluid medium container. As an optional feature, the system 200 furthermore may comprise a detection system 210 for detecting the liquid mixture sprayed through the nozzle and optionally also a feedback means 212 for providing feedback to a controller 214 of the manufacturing system 200 based on the output of the detection system. These components may be adapted for performing manufacturing of the beads as described in the second aspect. By way of example, a schematic manufacturing system is shown in Fig. 6.

## Claims

1. A method for manufacturing a lyophilized bead (7), the method comprising:
- providing a liquid mixture (2) having a freezing temperature, said liquid mixture (2) comprising:
- one or more magnetic or magnetisable particles (34), and
- an organic moiety,
- atomizing or spraying the liquid mixture (2) into a fluid medium (4) having a temperature lower than said freezing temperature, thus forming a bead (7), and
- freezing drying the bead (7).

2. The method of claim 0, wherein spraying the liquid mixture (2)comprises forming droplets (3) of said liquid mixture (2) and adding them into the fluid medium (4).

3. The method of any of claims 1 to 2, wherein spraying the liquid mixture comprises jetting droplets in a droplet stream into the fluid medium (4).

4. The method of any of claims 1 to 3, the method further comprising, functionally or geometrically influencing the liquid mixture (2) during the atomizing or spraying of the liquid mixture (2).

5. The method of claim 4, wherein functionally or geometrically influencing the liquid mixture comprises magnetically influencing said liquid mixture (2).

6. The method of any of claims 1 to 5, wherein the fluid medium (4) is a cryogenic liquid.

7. A bead (7) for use in bio-sensing, the bead (7) comprising:
- an organic moiety, and
- one or more magnetic or magnetisable particles (34),
- the bead being lyophilized.

8. The bead (7) according to claim 7, wherein the organic moiety comprises a matrix.

9. The bead (7) according to any of claims 7 to 8, wherein said organic moiety comprises any or a combination of one or more probes (32), one or more primers or one or more vectors.

10. The bead (7) according to claim 9, wherein said one or more probes (32) are immobilized on said one or more magnetic or magnetisable particles (34).

11. The bead (7) according to any of claims 9 to 10, wherein said one or more probes (32) are antibodies.

12. The bead (7) according to any of claims 7 to 11, wherein said organic moiety comprises any of a therapeutic or diagnostic or a binding agent or a carbohydrate or a proteinaceous material or a nucleotide sequence or a combination of any of these.

13. The bead (7) according to any of claims 7 to 12, wherein the organic moiety comprises one or more sugars (38) and/or the bead (7) comprises one or more salts (36).

14. The bead (7) according to claim 13, wherein said one or more sugars (38) are selected from the group consisting of mannitol, trehalose, sucrose and mixtures thereof.

15. The bead (7) according to any of claims 7 to 14, wherein the bead (7) has a diameter between 10 µm and 300 µm.

16. The bead (7) according to any of claims 7 to 15, wherein all magnetic dipoles of the magnetic or magnetisable particles (34) are oriented along a same predetermined orientation.

17. A process for analyzing the presence of an analyte in a sample fluid (10), said analyte being able to interact with an organic moiety, said process comprising:
- providing said sample fluid (10),
- contacting said sample fluid (10) with one or more beads (7), said one or more beads (7) comprising:
- one or more magnetic or magnetisable particles (34), and
- an organic moiety,
- the beads being lyophilized, and
- detecting the interaction between said sample fluid (10) and said organic moiety.

18. The process of claim 17, the organic moiety comprising one or more probes (32) being retained or immobilized on the one or more magnetic or magnetisable particles (34), wherein the step of detecting comprises detecting interaction between said sample fluid (10) and said one or more probes (32) by detecting said magnetic or magnetisable particles (34).

19. The process of claim 17 or claim 18, wherein the step of detecting comprises:
- providing a sensor surface (15) comprising analyte analogues immobilized thereon or therein, said analyte analogues being able to interact with said organic moiety,
- contacting said sample fluid contacted with said one or more beads (7) with said sensor surface (15), and
- detecting the presence of one or more magnetic or magnetisable particles (34) on or in said sensor surface (15).

20. The process of any of claims 17 to 19, wherein, prior to said contacting, said one or more beads (7) are located on or in said sensor surface (15).

21. The process of any of claims 17 to 20, wherein the step of contacting said sample fluid (10) with one or more beads (7) is performed in a first container (13) or channel separated from the sensor surface (15), said first container (13) or channel comprising said one or more beads (7) and being able to pass said sample fluid (10) to said sensor surface (15).

22. The process of claim 21, wherein the process comprises prior to said contacting, modifying said sample fluid (10) by contacting the sample fluid (10) with auxiliary beads (11) in a second container (14) or channel, said auxiliary beads (11) comprising one or more modifying agents, the beads being lyophilized, and thereafter providing the modified sample fluid to the first container (13) or channel.

23. The process of any of claims 17 to 22, wherein said analyte is a drug of abuse.

24. The process of any of claims 17 to 23, wherein said sample fluid comprises saliva.

25. A sensor system (100) comprising:
- one or more beads (7), said one or more beads (7) comprising:
- an organic moiety, and
- one or more magnetic or magnetisable particles,
- the beads being lyophilized, and
- a sensor surface (15).

26. A method for manufacturing a sensor system (100) comprising one or more beads (7), said one or more beads (7) comprising:
- an organic moiety and,
- one or more magnetic or magnetisable particles (34), the beads being lyophilized, said method comprising:
- providing a sensor surface (15), and
- providing said lyophilized beads (7) in said sensor system (100).

27. The method of claim 26, wherein said providing said lyophilized beads (7) is performed by magnetic actuation.

28. A system for manufacturing (200) a lyophilized bead (7) the system comprising, a container (202) for containing a liquid mixture (2) comprising the components of the lyophilized beads, an atomizing or spraying means (204) with an atomizing or spraying nozzle (5) for atomizing or spraying the liquid mixture (2) into a fluid medium (4) and a functionally and/or geometrically adjusting means (206) for adjusting the liquid mixture after leaving the spraying nozzle and prior to contacting the fluid medium (4).

29. A system for manufacturing (200) according to claim 28, wherein the functionally and/or geometrically adjusting means (206) is a magnetic or electromagnetic field generating means.

30. A system for manufacturing (200) according to any of claims 28 to 29, the system further comprising a detection means for visualizing the atomized or sprayed liquid mixture.
